# EUROPEAN PATENT SPECIFICATION

(11) **EP 3 378 458 B1**
(45) Date of publication and mention of the grant of the patent: **27.11.2019**
(21) Application number: 18382186.7
(22) Date of filing: 21.03.2018
(51) Int. Cl.: A61G 11/00, A47D 13/00, A47D 13/08, A61M 21/02

(54) **CONTAINMENT AND SENSORY STIMULATION SYSTEM FOR PREMATURE BABIES**
KASTEN UND SINNESSTIMULATIONSSYSTEM FÜR FRÜHGEBORENE
SYSTÈME DE CONFINEMENT ET DE STIMULATION SENSORIELLE POUR BÉBÉS PRÉMATURÉS

(30) Priority: 22.03.2017 ES 201730320 U
(43) Date of publication of application: 26.09.2018
(73) Proprietor: Hospital Sant Joan de Deu, 08950 Esplugues de Llobregat Barcelona (ES); Fundació Privada Elisava Escola Universitária, 08002 Barcelona (ES)
(72) Inventor: DÍAZ MECCHIA, Eva, 08950 ESPLUGUES DE LLOBREGAT (Barcelona) (ES); DEL CORRAL GONZÁLEZ, Ana María, 08002 BARCELONA (ES); GONZÁLEZ COLOMINAS, Marta, 08002 BARCELONA (ES); GARCIA FERNANDEZ, Miguel, 08950 ESPLUGUES DE LLOBREGAT (Barcelona) (ES)
(74) Representative: Herrero & Asociados, S.L.

(56) References cited:
- US-A1- 2008 092 300
- US-A1- 2011 144 416
- US-A1- 2014 330 070
- US-B1- 8 220 089

## Description

### Field of the invention

The present invention relates to a containment and sensory stimulation system for premature babies, applicable in the hospital setting in general, and especially for use in intensive care units (ICUs) for premature newborns.

### Background of the invention

The definition of premature baby is that of a newborn child of less than 37 weeks gestation and weighing less than 2500 g. Within this category, those babies with a weight of 1500 g or less fall under the group that deserves the greatest attention, both to ensure their survival, and to reduce the risk of disability in later stages of their lives.

Premature babies lack the body fat needed to maintain body temperature, even if they are well wrapped up with blankets. For this reason, incubators or radiant heaters are used to maintain their temperature. Maintaining the temperature of babies within the normal limits helps them to grow faster.

Incubators are usually made from transparent plastics and completely surround the baby's body to keep it warm, to reduce the chances of infection and to limit water loss. On the other hand, radiant heaters are open beds that are heated electrically. They are used when medical personnel need frequent access to the baby to provide the care they need.

Premature babies have special nutritional needs because they grow faster than full term babies and their digestive systems are immature. Breast milk is an excellent nutritional source, but premature babies are too immature to feed directly from the breast of the mother or the bottle until they have a gestational age of between 32 and 34 weeks. Most premature babies have to feed very slowly because of the risk of developing an intestinal infection exclusive to premature babies called necrotizing enterocolitis. The mother can express milk to be later administered to the baby by an intubation, i.e., a tube that is inserted through the baby's mouth or nose and reaches the stomach.

Another very important aspect to be taken into account in premature babies is their positioning during the time they spend in the incubator, that is, the position in which they are placed and the mobility applied to them. Such positioning is usually carried out through external elements (also called "nests"), which help give the baby the containment necessary to make it feel comfortable and safe. Also, both health personnel and parents should familiarise themselves with the positioning techniques of premature babies, being aware that good posture will form the basis for normal neuromotor development.

This is due to the fact that within the womb the normal motor development of a baby follows certain principles that determine that at the end of gestation the newborn has a flexor pattern that is the basis for its subsequent development. In other words, a full term baby presents a "fetal position" with its arms and legs tucked in or folded on its body and with its hands and arms tight. However, said flexor pattern is not fully developed in premature babies.

A correct positioning helps provide the baby with conditions that allow it to obtain said flexor pattern, preventing physical and neurological abnormalities. Thus, every time that the baby's position has to be changed, and depending on the characteristics it presents, it must be determined which is the most appropriate position to reduce risks. In addition, if it stays in the same position for a long time it may develop skin wounds, since, as it does not have any fat, that skin is in direct contact with the bones.

Thus, premature newborns may develop hip deformities, muscle abnormalities, loss of functionality, etc. Furthermore, as they are in incubators, said incorrect development affects them neurologically because it limits exploration of the environment, and therefore, reduces experience and learning. Also, these babies are characterised by being very stressed, due to the discomfort and the insecurity of being separated from the mother in a completely unknown environment (incubators).

Sensory stimulation in premature babies through the sensations that they have experienced during the months in the womb (reproduction of the mother's heartbeat, movements that emulate breathing, temperature, etc.), improves the babies' health stimulating them while relaxing them. Also, certain environmental conditions (faint light, silent environments, etc.) during the time they stay in incubators also allow for the reproduction of conditions similar to those they were used to in the mother's womb, thereby increasing their comfort and reducing their stress.

The document EP2659929A2 shows a sensory stimulation device for premature babies, based on a cradle that integrates an air chamber inside that emulates the shape of the lungs, and which, controlled by an external sensor connected to the mother, expands and contracts depending on her breathing and heartbeat.

One of the main problems such devices have, in addition to their complexity, is their lack of modularity. In particular, the part corresponding to the cradle is usually an indivisible element, consisting of an inseparable rest base and containment side, which integrate the sensory stimulation elements inside. This results in a more difficult device to manipulate, clean, repair and/or exchange, which has a negative impact on its usability.

US2011144416 A1 discloses an apparatus that simulates the movement an infant might experience while being held on its mother's chest is provided. The apparatus produces controlled and rhythmic motions similar to the expansion and contraction of an adult's chest or torso during normal breathing.

US8220089 B1 discloses a sleeper apparatus for holding an infant designed to simulate the feeling of being held in a caregiver's arms is herein disclosed. The sleeper is similar in shape to that of a canoe and is able to accommodate most infants via a removable insert section. The insert may be added or removed, depending on the size of the infant. The sleeper is made using padded foam materials and comprises an air bladder located along an inside wall of a perimeter enclosure.

The present invention solves the above problems through a containment and sensory stimulation system for premature babies as further disclosed in claim 1, consisting of multiple modular components separable from each other, easily interchangeable and removable, which can be used separately, and which facilitate their use by medical personnel and parents, allowing for convenient, intuitive and rapid use thereof. And in which, essentially, the correct position and stimulation of the baby is ensured at all times, so that its action is comforting, soothing and enriching for the baby's growth and development.

### Description of the invention

The containment and sensory stimulation system for premature babies of the present invention comprises:
- a rest and sensory stimulation base, whose rest surface for the baby's rest has a continuous upward and downward movement that resembles that of human breathing; and
- a containment side that surrounds perimetrally the rest and sensory stimulation base for the baby's containment.

The rest and sensory stimulation base and the containment side have a modular character, allowing the engagement and disengagement of both components by reusable joining elements.

The modular character of this system is of great importance, given that it facilitates its use by medical personnel and parents, optimising intervention times on the same, and thus, reducing the baby's stress. For example, the mother need only pick up the rest and sensory stimulation base on which the baby is lying and quietly place it on the chest. Once the baby is on the chest, said base can be removed from the baby's back and simply hold it with the hands or cover it with a blanket.

As for the configuration of the rest and sensory stimulation base, preferably this comprises a rest mattress on which the rest surface is located; and a sensory mattress base on which the rest mattress is arranged. The concept of modularity is also maintained in this case, given that the rest mattress is simply supported on the sensory mattress base without fixed attachment means, so that it achieves a very dynamic disassembly and usability, which favours the interchangeable and removable character of these components.

The rest mattress is preferably of viscoelastic polyurethane foam (for example, of 55 kg/cc) of soft touch and shape memory *(memory foam),* which is arranged in a cotton cover that has a waterproof layer to prevent the polyurethane ageing. This allows the rest mattress to adapt perfectly to the shape of the baby providing comfort, but at the same time preventing the baby from noticing the components lying under the mattress when a pressure is applied thereon (improving the baby's circulation and avoiding pressure ulcers). This pressure is uniformly dissipated overall the surface, avoiding that high pressure is produced from the different points of contact. Also, the containment side is preferably of polyurethane foam (e.g., 30 kg/cc) due to its stability and ease of cutting.

In turn, the sensory mattress base consists of a base or mattress support, delimited at the top by a sheet arranged under the rest mattress in contact with it, defining between said mattress base and said sheet a technical chamber in which a motorised cam mechanism capable of producing the upward and downward movement on the sheet is located. This continuous "human breathing" (breathing pattern) movement allows to simulate the baby's maternal environment to stimulate it and relax it at the same time.

To carry out such movement, preferably the motorised cam mechanism comprises a first micro geared motor allowing the rotation of a first cam; and a second micro geared motor allowing the rotation of a second cam; wherein said cams are arranged in a central part of the sensory mattress base in contact with a central part of the sheet. Micro geared motors are responsible for creating the cycles of the breathing pattern, and the sheet goes up and down thanks to the "input" given by the cam eccentric load. This mechanism is regulated with a potentiometer. The breathing pattern is preferably 12 inspirations per minute (accepted as good by professionals), simulating quiet human breathing.

An example of the type of micro geared motors to be used may be: a micro geared motor measuring approx. 24 x 10 x 12 mm, with 10 g weight, 3 mm axis diameter, 298:1 gear ratio, 3 to 9 Vcc design voltage, 45 rpm rotational speed at no load, 40 mA (Max: 360 mA) consumption at no load, and 27,9 gr/mm (25 oz/in) (max) torque (force). The sheet is preferably of methacrylate (PMMA), with sufficient stiffness to prevent it from being bent by the baby's weight, but at the same time, with sufficient flexibility to allow it to go up and down through the action of the micro geared motors.

The rest and sensory stimulation base comprises an enveloping cover that laterally envelops the rest mattress and sensory mattress base, and also has:
- a first containment strap which transversely enfolds the containment side at a first zone thereof allowing the wrapping of the baby's arms, chest or back; and
- a second containment strap, wider than the first containment strap, which transversely enfolds the containment side at a second zone thereof allowing the wrapping of the baby's legs.

The enveloping cover is preferably of knitted cotton which is characterised by being very breathable, soft and very adaptable to shapes. Cotton is a perfectly washable material at high temperatures, allowing the cover to be washed frequently. In addition, it has a percentage of elastane, which gives it flexibility, making its placement and adaptation to different outlines easier. The fact that the cover is breathable is important as it contacts directly the baby's skin. In turn, given that the cradle of the present invention is intended to be used in an incubator, where the environment is characterised by being warm, if it were a product that is lined with a non-breathable material that would not be good for the baby's health. The fact that the cover is soft is important, as premature babies have very sensitive and wrinkled skin. The fact that the cover is knitted helps it to adapt very well to foam shapes, avoiding wrinkles. Finally, the cover is semi-permeable to protect the viscoelastic foam from possible fluids.

Preferably, the containment straps are removable, being able to be fastened to each side of the cover by using buttons, or other similar, quick, durable, and aesthetic joining elements.

As for the design and forms of the main components of the system, preferably the rest and sensory stimulation base and the containment side have perimetrally a matching elliptical outline. Also, these components can also take other organic or natural forms, which in addition to giving it a harmonious and original look, seek to adapt to the shape of the hand in the simplest way.

Also, preferably, the containment side has an undulating upper profile which defines longitudinally four segments of said containment side:
- a first segment in which the undulating profile has a first height configured to exceed the baby's head;
- a second segment in which the undulating profile descends from the first height to a second height configured to leave the baby's face uncovered, and ascends from that second height to a third height coinciding with the first height;
- a third segment in which the undulating profile ascends from the third height to a fourth height configured to cover the baby's trunk and legs, and descends from that fourth height to a fifth height coinciding with the third height; and
- a fourth segment in which the undulating profile descends from the fifth height to a sixth height configured to enable the baby to stretch its legs.

Reusable joining elements can be selected from zips, buttons, fittings, hooks, velcro® type quick fasteners, or other similar elements. According to a preferred case, reusable joining elements comprise:
- a first zip band following the upper outline of the rest and sensory stimulation base; and
- a second zip band following the bottom outline of the containment side, cooperating with the first zip band to allow the engagement and disengagement of both components.

The containment and sensory stimulation system for premature babies of the present invention further comprises a series of complements or accessories to further promote correct postural positioning. Its function is to avoid the "flattening" of some parts of the body over the rest mattress, maintaining the flexion and the midline thereof. In addition, all of these components maintain the concept of modularity previously mentioned, being easily assembled and dissembled to operate independently or in combination.

In this sense, the containment and sensory stimulation system for premature babies of the present invention comprises a first rest complement, called "prono", configured to be arranged on the rest and sensory stimulation base internally fitted to the containment side. This first rest complement allows to position the baby in the prone position, with its arms collected within the containment side.

To do this, preferably the first rest complement consists of:
- a first portion of semicircular outline, with a first support surface descending to a first end, to support the baby's head;
- a second portion of rectangular outline, with a second horizontal support surface, for supporting the baby's chest; and
- a third portion that expands laterally into two symmetrical sub-portions that have a curved recess with a third support surface that descends to said curved recess, to support the baby's knees.

The slopes should be pronounced in the area of the abdomen and the head, obtaining the baby's flexion and the benefits that this brings. For the first rest complement, preferably, viscoelastic polyurethane foam (for example, 40 kg/cc) is used, which returns to its original shape faster than the rest mattress, but which also fits perfectly into different shapes because it is very soft.

The containment and sensory stimulation system for premature babies of the present invention comprises a second "large" cylindrically configured rest complement. This second rest complement allows positioning the baby in the lateral decubitus position, placing said "large" cylinder between the baby's arms and knees, and held to its chest, to maintain the midline, and to prevent the arm or leg from acquiring bad posture through the force of gravity.

The containment and sensory stimulation system for premature babies of the present invention comprises a third "small" cylindrically configured rest complement. This third rest complement is combined with the first rest complement in the prone position. For this, the "small" cylinder is placed under the ankles, preventing them from remaining flat on the rest mattress and obtaining an angle between the tibia and the foot.

The second rest complement and the third rest complement may be used together in the supine position. For this, the "small" cylinder is placed on the rest mattress under the baby's nape or neck to prevent its chin from getting over close to the neck, otherwise the baby would acquire bad physical posture and have difficulty in passing fluids through its throat. In this same position, the "large" cylinder is placed under the knees, helping flexion of the legs, and thus, a contained position.

Preferably, cylindrical complements are of polyester fibre and axially hollow, of pleasant touch, breathable, and with good cushioning. Easily washable and recyclable.

### Brief description of the drawings

A series of drawings that help to better understand the invention and which are expressly related to a preferred embodiment of said invention and are presented as a non-limiting examples thereof is briefly described below.
Figure 1 represents a perspective view of the containment and sensory stimulation system for premature babies of the present invention.
Figure 2 represents a perspective exploded view of Figure 1.
Figure 3 represents a plan view of Figure 1.
Figure 4 represents a plan view of Figure 1, without showing the rest complements.
Figure 5 represents a sectioned view of the containment and sensory stimulation system for premature babies according to the section line A-A of Figure 4.
Figure 6 represents a lower profile view of the deployed containment side.
Figure 7 represents a sectioned view of the containment side according to the section line B-B of Figure 6.
Figure 8 represents a perspective exploded view of the rest and sensory stimulation base.
Figure 9 represents a plan view of the rest and sensory stimulation base, without showing the enveloping cover.
Figure 10 represents a sectioned view of the rest and sensory stimulation base according to the section line C-C of Figure 9.
Figure 11 represents a perspective exploded view of the sensory mattress base.
Figure 12 represents a plan view of the sensory mattress base.
Figure 13 represents a sectioned view of the sensory mattress base according to the section line D-D of Figure 12.
Figure 14 represents a sectioned view of the sensory mattress base according to the section line E-E of Figure 12.
Figure 15 represents a perspective exploded view of the motorised cam mechanism.
Figure 16 represents a plan view of the first rest complement.
Figure 17 represents an elevation view of Figure 16.
Figure 18 represents a lower view of Figure 16.
Figure 19 represents an elevation view of the second rest complement.
Figure 20 represents an elevation view of the third rest complement.
Figures 21A, 21B and 21C show a first mode of application of the containment and sensory stimulation system for premature babies of the present invention corresponding to the prone position.
Figures 22A, 22B and 22C show a second mode of application of the containment and sensory stimulation system for premature babies of the present invention corresponding to the lateral decubitus position.
Figures 23A, 23B and 23C show a third mode of application of the containment and sensory stimulation system for premature babies of the present invention, corresponding to the supine position.

### Detailed description of the invention

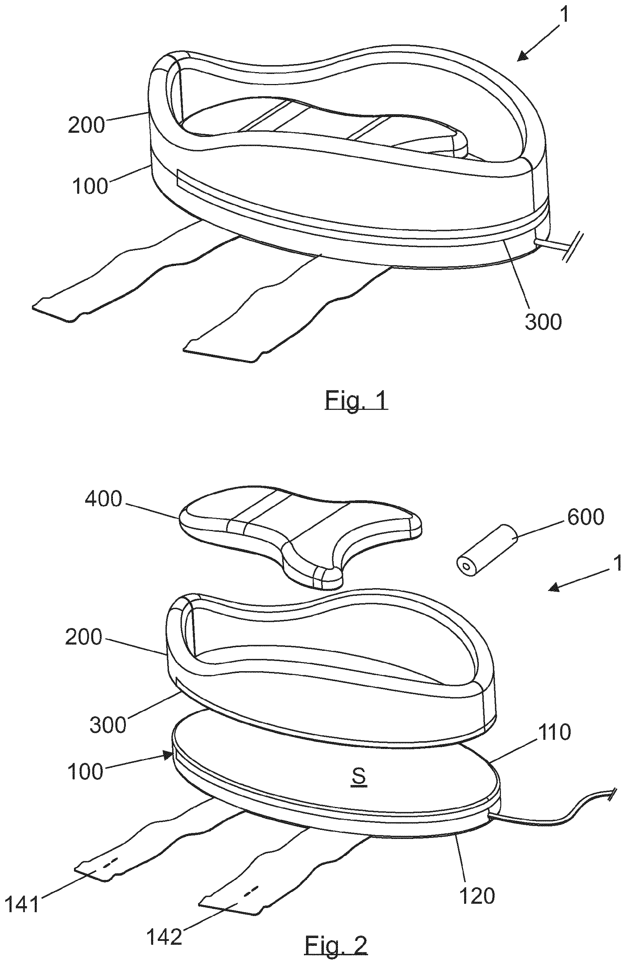

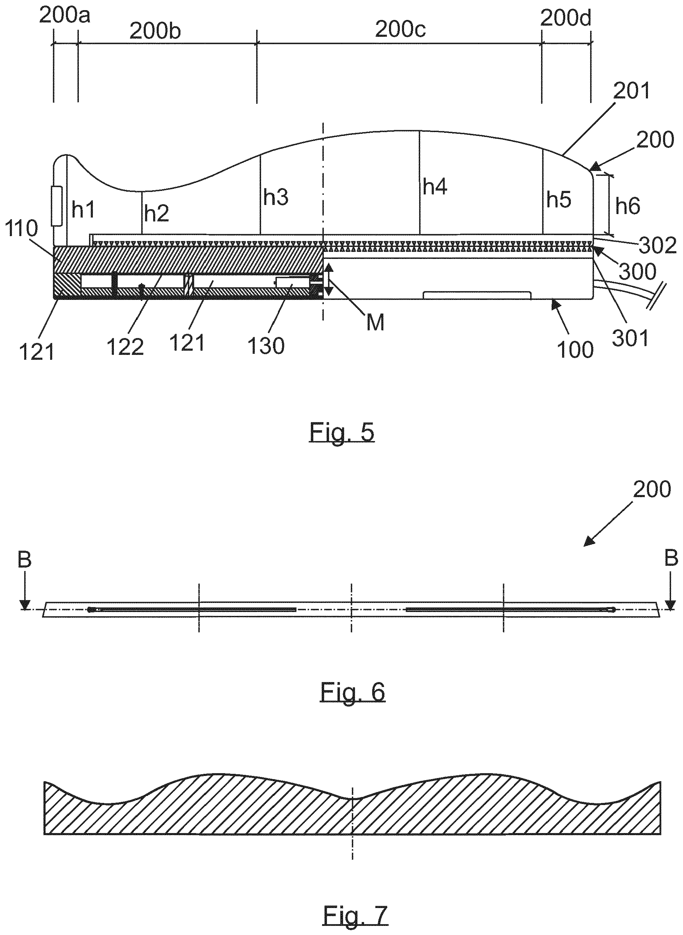

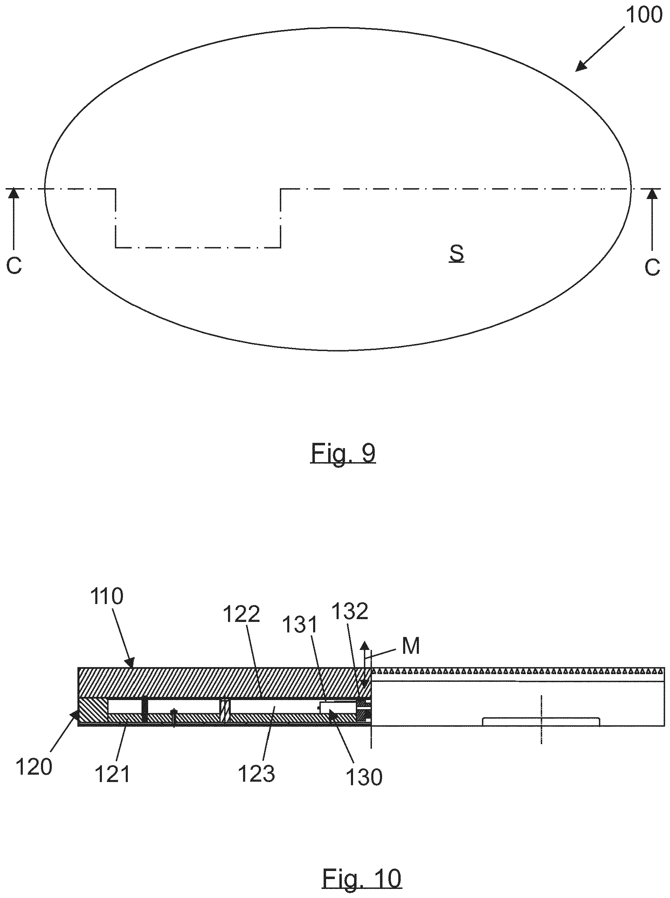

Figures 1 - 3 show various views of the containment and sensory stimulation system for premature babies of the present invention. As can be seen, said system (1) comprises:
- a rest and sensory stimulation base (100), whose rest surface (S) for the baby's rest has a continuous upward and downward movement (M) that resembles human breathing, Figures 5 and 10; and
- a containment side (200) that perimetrally surrounds the rest and sensory stimulation base (100) for the baby's containment.

The rest and sensory stimulation base (100) and the containment side (200 have a modular character that allows the engagement and disengagement of both components (100, 200) by reusable joining elements (300).

The rest and sensory stimulation base (100) and the containment side (200) have perimetrally a matching elliptical outline.

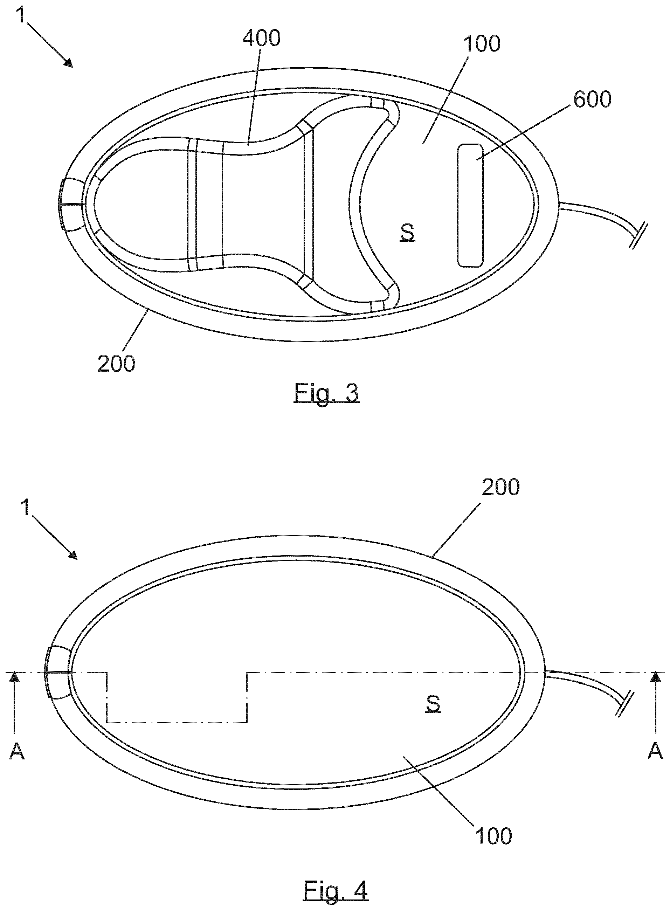

Figures 4 and 5 respectively show a plan view and a sectioned view of the containment and sensory stimulation system for premature babies, without showing the rest complements (400, 500, 600).

As can be seen, the containment side (200) has an undulating upper profile (201) which defines longitudinally four segments (200a, 200b, 200c, 200d) of the containment side (200):
- a first segment (200a) in which the undulating profile (201) has a first height (h1) configured to exceed the baby's head;
- a second segment (200b) in which the undulating profile (201) descends from the first height (h1) to a second height (h2) configured to leave the baby's face uncovered, and ascends from said second height (h2) to a third height (h3) coinciding with the first height (h1);
- a third segment (200c) in which the undulating profile (201) ascends from the third height (h3) to a fourth height (h4) configured to cover the baby's trunk and legs, and descends from said fourth height (h4) to a fifth height (h5) coinciding with the third height (h3); and
- a fourth segment (200d) in which the undulating profile (201) descends from the fifth height (h5) to a sixth height (h6) configured to enable the baby to stretch its legs.

The reusable joining elements (300) comprise:
- a first zip band (301) following the upper outline of the rest and sensory stimulation base (100); and
- a second zip band (302) following the lower outline of the containment side, cooperating with the first zip band (301) to allow the engagement and disengagement of both components (100, 200).

Figures 6 and 7 respectively show a lower profile view and a sectioned view of the containment side (200), when it is deployed. As can be seen, the lower face comprises opening and closing means (210), of the zip type, prepared for the extraction of the polyurethane foam contained inside for its washing or replacement.

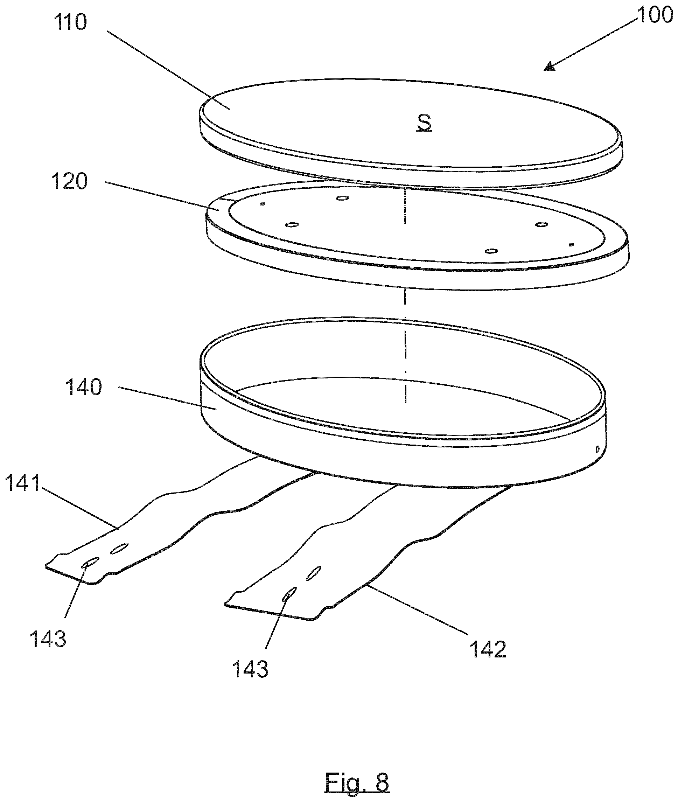

Figures 8 and 9 respectively show a perspective exploded view and a plan view of the rest and sensory stimulation base (100). As can be seen, it comprises a rest mattress (110) on which the rest surface (S) is located; and a sensory mattress base (120) on which the rest mattress is arranged (110). The concept of modularity is also maintained in this case, since the rest mattress (110) is simply supported on the sensory mattress base (120) without fixed attachment means, so that a very dynamic disassembly and usability is achieved, which favours the interchangeable and removable character of these components.

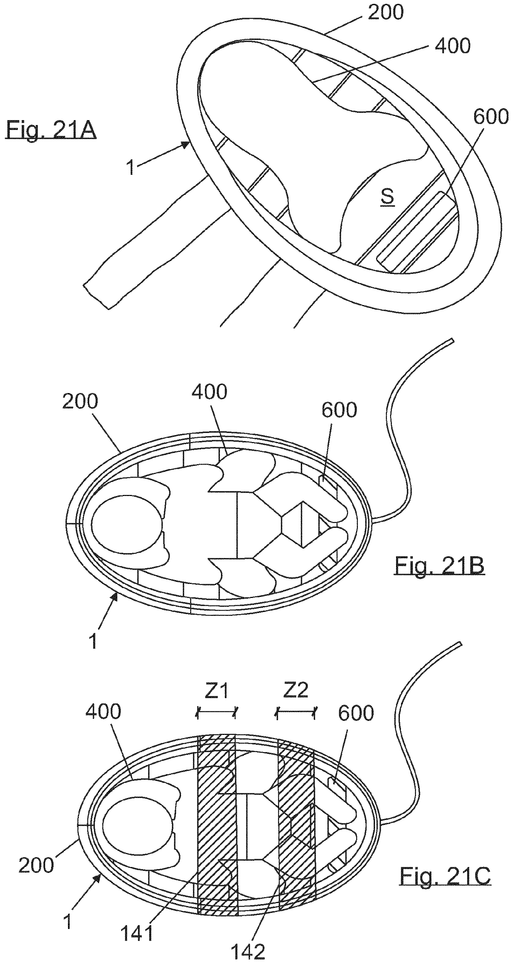

The rest and sensory stimulation base (100) comprises an enveloping cover (140) laterally enveloping the rest mattress (110) and the sensory mattress base (120), and also has:
- a first containment strap (141) which transversely enfolds the containment side (200) at a first zone (Z1) thereof allowing the wrapping of the baby's arms, chest or back, Figures 21c, 22c and 23c; and
- a second containment strap (142), larger than the first containment strap (141), which transversely enfolds the containment side (200) at a second zone (Z2) thereof allowing the wrapping of the baby's legs, Figures 21c, 22c and 23c.

The containment straps (141, 142) are removable, and can be fastened to either side of the enveloping cover (140) by quick joining elements (143), buttons in this example.

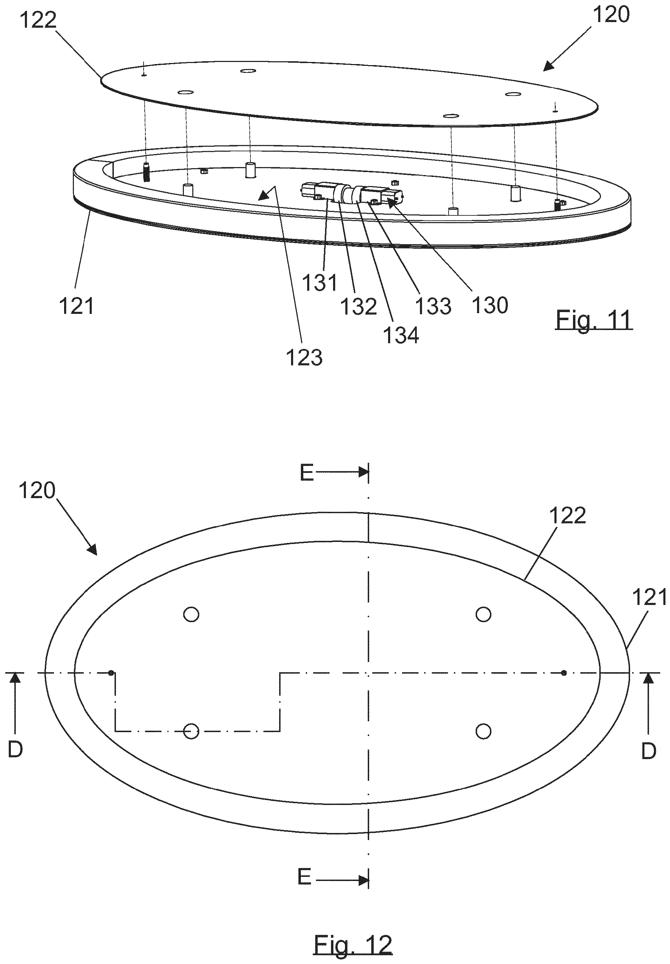

As can be seen in Figures 10-14, the sensory mattress base (120) consists of a base or mattress base support (121), delimited at the top by a sheet (122) arranged under the rest mattress (110) in contact with it, defining between said mattress base (121) and said sheet (122) a technical chamber (123) in which a motorised cam mechanism (130) capable of producing the upward and downward movement (M) on the sheet (122) is located. This continuous "human breathing" (breathing pattern) movement allows to simulate the baby's maternal environment to stimulate it and relax it at the same time.

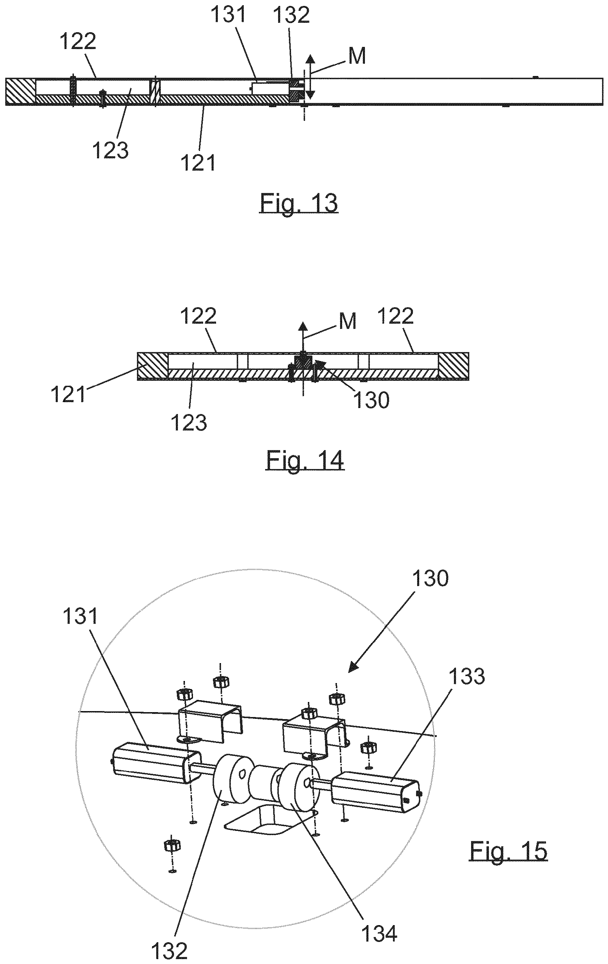

Figure 15 shows the motorised cam mechanism (130) in greater detail. As can be seen, said motorised cam mechanism (130) comprises a first micro geared motor (131) which allows the rotation of a first cam (132); and a second micro geared motor (133) allowing the rotation of a second cam (134); wherein said cams (132, 134) are arranged in a central part of the sensory mattress base (120) in contact with a central part of the sheet (122). The micro geared motors (131, 133) are responsible for creating the cycles of the breathing pattern, and the sheet (122) goes up and down thanks to the "input" given by the eccentric load of the cams (132, 134).

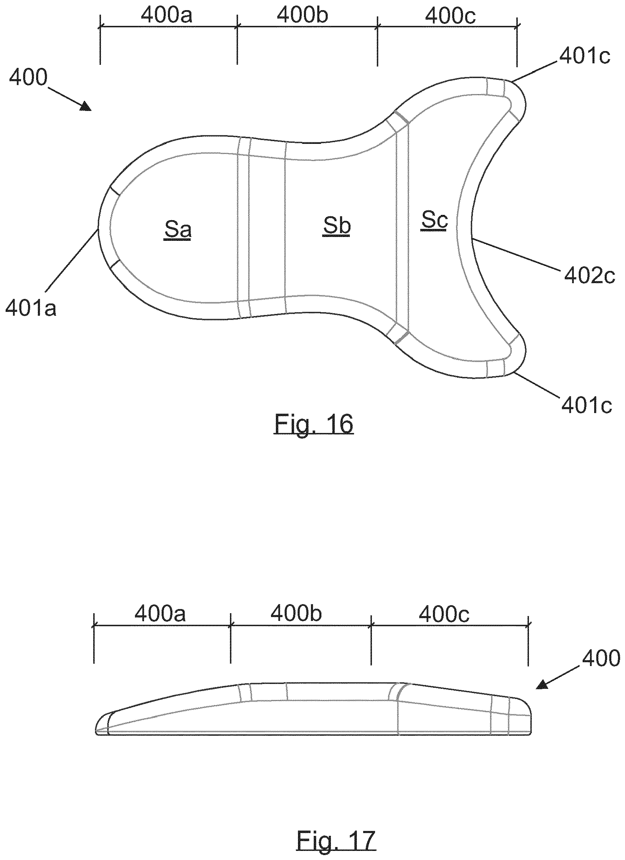

Figures 16-18 show various views of the first rest complement (400). Said first rest complement (400), called "prono", is configured to be arranged on the rest and sensory stimulation base (100) internally fitted to the containment side (200), Figures 1-3.

The first rest complement (400) consists of:
- a first portion (400a) of substantially semicircular outline, with a first support surface (Sa) descending towards a first end (401 a), for support of the baby's head;
- a second portion (400b) of rectangular outline with a second horizontal support surface (Sb), to support the baby's chest; and
- a third portion (400c) that expands laterally into two symmetrical sub-portions (401 c) with a curved recess (402c), with a third support surface (Sc) that descends into said curved recess (402c), to support the baby's knees.

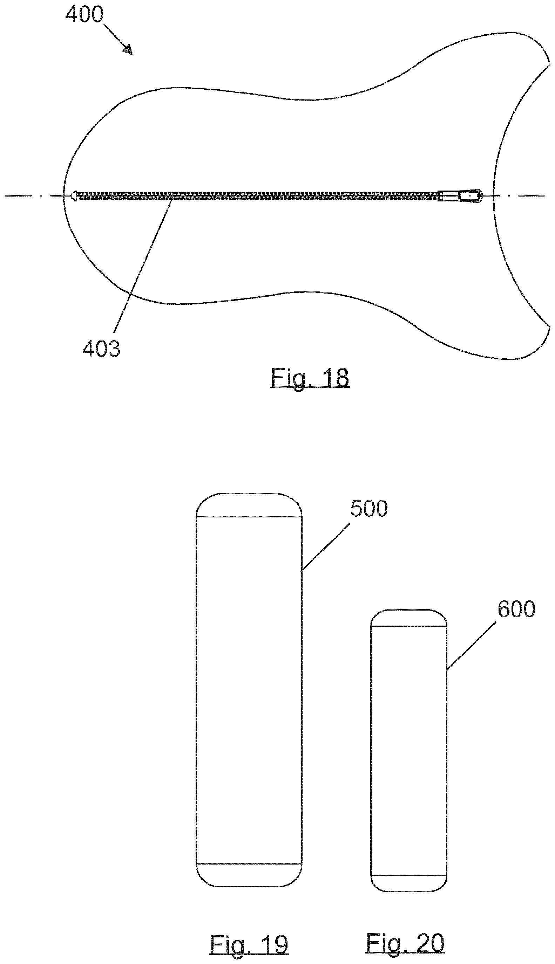

Figure 19 shows an elevation view of the second rest complement (500) of "large" cylindrical configuration.

Figure 20 shows an elevation view of a third rest complement (600) of "small" cylindrical configuration.

As can be seen in Figures 19 and 20, the second rest complement (500) and the third rest complement (600) are of different sizes.

Figures 21A, 21B and 21C show a first mode of application of the containment and sensory stimulation system for premature babies of the present invention corresponding to the prone position. As can be seen, the first rest complement (400) allows positioning the baby in the prone position with its arms collected within the containment side (200), combining it with the third rest complement (600), which is placed under the baby's ankles, preventing them from remaining flat on the rest mattress (110) and obtaining an angle between the tibia and the foot.

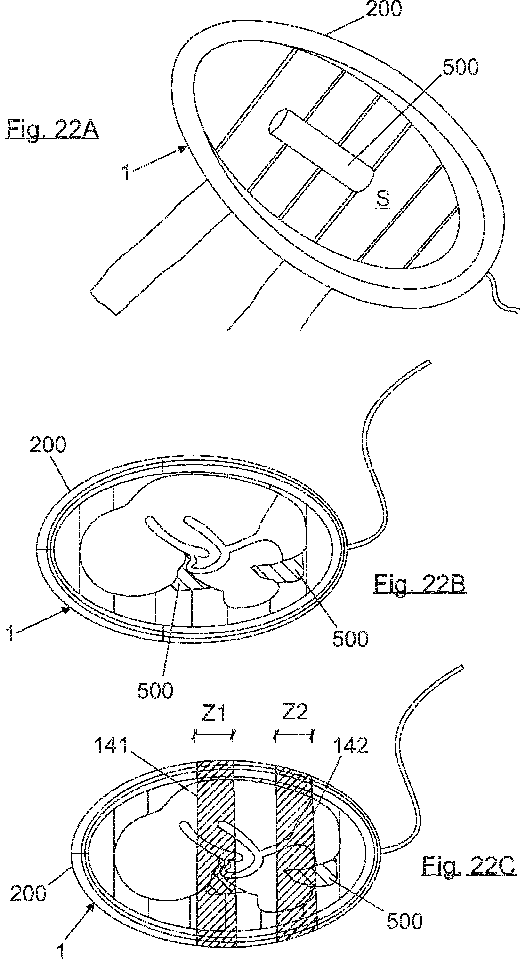

Figures 22A, 22B and 22C show a second mode of application of the containment and sensory stimulation system for premature babies of the present invention corresponding to the lateral decubitus position. This second rest complement (500) allows positioning the baby in the lateral decubitus position, placing said "large" cylinder between the arms and knees of the baby, and held to its chest to maintain the midline and to prevent the arm or leg from acquiring bad posture.

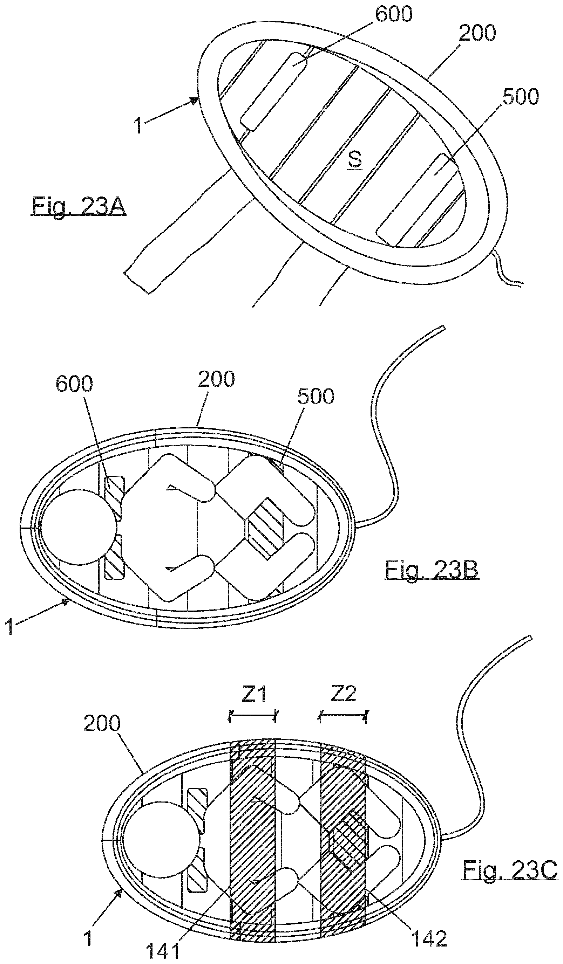

Figures 23A, 23B and 23C show a third mode of application of the containment and sensory stimulation system for premature babies of the present invention, corresponding to the supine position. In this case, the second rest complement (500) and the third rest complement (600) are used together in the supine position. For this, the "small" cylinder (600) is placed on the rest mattress (110) under the baby's nape or neck to prevent its chin from getting over close to the neck, otherwise the baby would acquire bad physical posture and have difficulty in passing fluids through its throat. In this same position, the "large" cylinder (500) is placed under the knees, helping flexion of the legs and thus contained position.

## Claims

1. A containment and sensory stimulation system for premature babies, said system (1) comprising:
- a rest and sensory stimulation base (100), whose rest surface (S) for the baby's rest has an upward and downward movement (M); and
- a containment side (200) which perimetrally surrounds the rest and sensory stimulation base (100) for the baby's containment;
- reusable joining elements (300);
wherein said rest and sensory stimulation base (100) and said containment side (200) have a modular character allowing the engagement and disengagement of both components (100, 200) by said reusable joining elements (300), **characterised in that** the rest and sensory stimulation base (100) comprises a rest mattress (110) on which the rest surface (S) is located; and a sensory mattress base (120) on which the rest mattress is arranged (110), and
**in that** the rest and sensory stimulation base (100) also comprises an enveloping cover (140) laterally enveloping the rest mattress (110) and the sensory mattress base (120).

2. The containment and sensory stimulation system for premature babies according to claim 1, **characterised in that** the sensory mattress base (120) consists of a mattress base support (121), delimited at the top by a sheet (122) arranged under the rest mattress (110) in contact with it defining between said mattress base support (121) and said sheet (122) a technical chamber (123) in which a motorised cam mechanism (130) capable of producing the upward and downward movement (M) on the sheet (122) is located.

3. The containment and sensory stimulation system for premature babies according to claim 2, **characterised in that** the motor cam mechanism (130) comprises a first micro geared motor (131) allowing the rotation of a first cam (132); and a second micro geared motor (133) allowing the rotation of a second cam (134); wherein said cams (132, 134) are arranged in a central part of the sensory mattress base (120) in contact with a central part of the sheet (122).

4. The containment and sensory stimulation system for premature babies according to any of claims 2 to 4, **characterised in that** the rest and sensory stimulation base (100) also has:
- a first containment strap (141) enfolding the containment side (200) at a first zone (Z1) thereof, allowing the wrapping of the baby's arms, chest or back; and
- a second containment strap (142), wider than the first containment strap (141), which transversely enfolds the containment side (200) at a second zone (Z2) thereof, allowing the wrapping of the baby's legs.

5. The containment and sensory stimulation system for premature babies according to any of claims 1 to 4, **characterised in that** the rest and sensory stimulation base (100) and the containment side (200) have perimetrally a matching elliptical outline.

6. The containment and sensory stimulation system for premature babies according to any of claims 1 to 5, **characterised in that** the containment side (200) has an undulating upper profile (201) which defines longitudinally four sections (200a, 200b, 200c, 200d) of the containment side (200):
- a first segment (200a) in which the undulating profile (201) has a first height (h1) configured to exceed the baby's head;
- a second segment (200b) in which the undulating profile (201) descends from the first height (h1) to a second height (h2) configured to leave the baby's face uncovered, and ascends from said second height (h2) to a third height (h3) coinciding with the first height (h1);
- a third segment (200c) in which the undulating profile (201) ascends from the third height (h3) to a fourth height (h4) configured to cover the baby's trunk and legs, and descends from said fourth height (h4) to a fifth height (h5) coinciding with the third height (h3); and
- a fourth segment (200d) in which the undulating profile (201) descends from the fifth height (h5) to a sixth height (h6) configured to enable the baby to stretch its legs.

7. The containment and sensory stimulation system for premature babies according to any of claims 1 to 6, **characterised in that** the reusable joining elements 300 are selected from zips, buttons, fittings, hooks, and velcro® type quick fasteners.

8. The containment and sensory stimulation system for premature babies according to any of claims 1 to 7, **characterised in that** the reusable joining elements (300) comprise:
- a first zip band (301) following the upper outline of the rest and sensory stimulation base (100); and
- a second zip band (302) following the lower outline of the containment side, cooperating with the first zip band (301) to allow the engagement and disengagement of both components (100, 200).

9. The containment and sensory stimulation system for premature babies according to any of claims 1 to 8, **characterised in that** it comprises a first rest complement (400) configured to be arranged on the rest and sensory stimulation base (100) internally fitted to the containment side (200).

10. The containment and sensory stimulation system for premature babies according to claim 9, **characterised in that** the first rest complement (400) consists of:
- a first portion (400a) of semicircular outline, with a first support surface (Sa) that descends to a first end (401 a), to support the baby's head;
- a second portion (400b) of rectangular outline with a second horizontal support surface (Sb), to support the baby's chest; and
- a third portion (400c) that expands laterally into two symmetrical sub-portions (401 c) with a curved recess (402c), with a third support surface (Sc) that descends into said curved recess (402c), to support the baby's knees.

11. The containment and sensory stimulation system for premature babies according to any of claims 1 to 10, **characterised in that** it comprises a second rest complement (500) of cylindrical configuration.

12. The containment and sensory stimulation system for premature babies according to any of claims 1 to 11, **characterised in that** it comprises a third rest complement (600) of cylindrical configuration.

13. The containment and sensory stimulation system for premature babies according to claims 11 and 12, **characterised in that** the second rest complement (500) and the third rest complement (600) are of different sizes.

## Patentansprüche

1. System zur Bewahrung und sensorischen Stimulation von Frühgeborenen, wobei das System (1) aufweist:
- ein Basiselement zum Liegen und zur sensorischen Stimulation (100), dessen Liegefläche (S) für die Ruhelage des Säuglings über eine Aufwärts- und Abwärts-Bewegung (M) verfügt; und
- eine Bewahrungsseite (200), die das Basiselement zum Liegen und zur sensorischen Stimulation (100) zum sicheren Bewahren des Säuglings in Umfangsrichtung umgibt;
- wiederverwendbare Verbindungselemente (300);
wobei das Basiselement zum Liegen und für sensorische Stimulation (100) und die Bewahrungsseite (200) modularen Charakter besitzen, der das Koppeln und Entkoppeln beider Komponenten (100, 200) durch die wiederverwendbaren Verbindungselemente (300) ermöglicht,
**dadurch gekennzeichnet, dass**
das Basiselement zum Liegen und für sensorische Stimulation (100) eine Liegematte (110), auf der die Liegefläche (S) angeordnet ist; und ein sensorisches Mattenbasiselement (120) aufweist, auf welchem die Liegematte (110) angeordnet ist,
und
das Basiselement zum Liegen und für sensorische Stimulation (100) ferner eine umhüllende Abdeckung (140) aufweist, die die Liegematte (110) und das sensorische Mattenbasiselement (120) seitlich umschließt.

2. System zur Bewahrung und sensorischen Stimulation für Frühgeborene nach Anspruch 1, **dadurch gekennzeichnet, dass** das sensorische Mattenbasiselement (120) aus einer Mattenbasiselementhalterung (121) besteht, die an der Oberseite durch eine Platte (122) begrenzt ist, die unter der Liegematte (110) und in Kontakt damit angeordnet ist, die zwischen der Mattenbasiselementhalterung (121) und der Schicht (122) eine Technikkammer (123) begrenzt, in der ein motorisierter Nockenmechanismus (130) liegt, der in der Lage ist, die Aufwärts- und Abwärtsbewegung (M) auf der Platte (122) zu erzeugen.

3. System zur Bewahrung und sensorischen Stimulation für Frühgeborene nach Anspruch 2, **dadurch gekennzeichnet, dass** der motorisierte Nockenmechanismus (130) einen ersten Mikro-Getriebemotor (131), der die Drehung einer ersten Nocke (132) ermöglicht; und einen zweiten Mikro-Getriebemotor (133) aufweist, der eine Drehung einer zweiten Nocke (134) ermöglicht; wobei die Nocken (132, 134) in einem zentralen Teil des sensorischen Mattenbasiselement (120) in Kontakt mit einem zentralen Teil der Platte (122) angeordnet sind.

4. System zur Bewahrung und sensorischen Stimulation für Frühgeborene nach einem der Ansprüche 2 bis 4, **dadurch gekennzeichnet, dass** das Basiselement zum Liegen und für sensorische Stimulation (100) ferner aufweist:
- einen ersten Haltegurt (141), der die Bewahrungsseite (200) an einer ersten Zone (Z1) einhüllt, sodass das Umschlingen der Arme, der Brust oder des Rückens des Säuglings möglich ist; und
- einen zweiten Haltegurt (142), der weiter als der erste Haltegurt (141) ist und der die Bewahrungsseite (200) an einer zweiten Zone (Z2) quer einhüllt, sodass das Umschlingen der Beine des Säuglings möglich ist.

5. System zur Bewahrung und sensorischen Stimulation für Frühgeborene nach einem der Ansprüche 1 bis 4, **dadurch gekennzeichnet, dass** das Basiselement zum Liegen und für sensorische Stimulation (100) und die Bewahrungsseite (200) ein Umfangsrichtung einen übereinstimmenden elliptischen Umriss haben.

6. System zur Bewahrung und zur sensorischen Stimulation für Frühgeborene nach einem der Ansprüche 1 bis 5, **dadurch gekennzeichnet, dass** die Bewahrungsseite (200) ein welliges oberes Profil (201) hat, das in Längsrichtung vier Abschnitte (200a, 200b, 200c, 200d) der Bewahrungsseite (200) begrenzt:
- ein erstes Segment (200a), in welchem das wellige Profil (201) eine erste Höhe (h1) hat, die so gestaltet ist, dass sie den Kopf des Säuglings übersteigt;
- ein zweites Segment (200b), in welchem das wellige Profil (201) von der ersten Höhe (h1) auf eine zweite Höhe (h2) absinkt, die geeignet ist, das Gesicht des Säuglings unbedeckt zu lassen, und von der zweiten Höhe (h2) zu einer dritten Höhe (h3) ansteigt, die mit der ersten Höhe (h1) übereinstimmt;
- ein drittes Segment (200c), in welchem das wellige Profil (201) von der dritten Höhe (h3) auf eine vierte Höhe (h4) ansteigt, die geeignet ist, den Rumpf und die Beine des Säuglings zu bedecken, und von der vierten Höhe (h4) auf eine fünfte Höhe (h5) absinkt, die mit der dritten Höhe (h3) übereinstimmt; und
- ein viertes Segment (200d), in welchem das wellige Profil (201) von der fünften Höhe (h5) auf eine sechste Höhe (h6) absinkt, die geeignet ist, dem Säugling zu ermöglichen, seine Beine auszustrecken.

7. System zur Bewahrung und sensorischen Stimulation für Frühgeborene nach einem der Ansprüche 1 bis 6, **dadurch gekennzeichnet, dass** die wiederverwendbaren Verbindungselemente (300) ausgewählt sind aus Reißverschlüssen, Knöpfen, Kupplungsstücken, Haken und Klett-Schnellverschlüssen.

8. System zur Bewahrung und sensorischen Stimulation für Frühgeborene nach einem der Ansprüche 1 bis 7, **dadurch gekennzeichnet, dass** die wiederverwendbaren Verbindungselemente (300) aufweisen:
- ein erstes Reißverschlussband (301), das dem oberen Umriss des Basiselements zum Liegen und zur sensorischen Stimulation (100) nachgeführt ist; und
- ein zweites Reißverschlussband (302), das dem unteren Umriss der Bewahrungsseite nachgeführt ist und mit dem ersten Reißverschlussband (301) so zusammenwirkt, dass ein Koppeln und Entkoppeln beider Komponenten (100, 200) möglich ist.

9. System zur Bewahrung und sensorischen Stimulation für Frühgeborene nach einem der Ansprüche 1 des 8, **dadurch gekennzeichnet, dass** es ein erstes Liegegegenstück (400) aufweist, das ausgebildet ist, auf dem Basiselement zum Liegen und zur sensorischen Stimulation (100), das intern mit der Bewahrungsseite (200) verbunden ist, angeordnet zu werden.

10. System zur Bewahrung und sensorischen Stimulation für Frühgeborene nach Anspruch 9, **dadurch gekennzeichnet, dass** das erste Liegegegenstück (400) besteht aus:
- einem ersten Bereich (400a) mit halbkreisförmigen Umriss und mit einer ersten Stützfläche (Sa), die zu einem ersten Ende (401a) hin abfällt, um den Kopf des Säuglings zu stützen;
- einem zweiten Bereich (400b) mit rechteckigem Umriss und mit einer zweiten horizontalen Stützfläche (Sb) zum Stützen der Brust des Säuglings; und
- einem dritten Bereich (400c), der sich lateral zu zwei symmetrischen Unterbereichen (401c) mit einer gekrümmten Vertiefung (402c) erweitert und eine dritte Stützfläche (Sc) hat, die in die gekrümmte Vertiefung (402c) absinkt, um die Knie des Säuglings zu stützen.

11. System zur Bewahrung und sensorischen Stimulation für Frühgeborene nach einem der Ansprüche 1 des 10, **dadurch gekennzeichnet, dass** es ein zweites Liegegegenstück (500) mit zylindrischem Aufbau aufweist.

12. System zur Bewahrung und sensorischen Stimulation für Frühgeborene nach einem der Ansprüche 1 bis 11, **dadurch gekennzeichnet, dass** es ein drittes Liegegegenstück (600) mit zylindrischem Aufbau aufweist.

13. System zur Bewahrung und sensorischen Stimulation für Frühgeborene nach Anspruch 11 und 12, **dadurch gekennzeichnet, dass** das zweite Liegegegenstück (500) und das dritte Liegegegenstück (600) unterschiedliche Größen haben.

## Revendications

1. Système de confinement et de stimulation sensorielle pour bébés prématurés, ledit système (1) comprenant :
- une base de repos et de stimulation sensorielle (100), dont une surface de repos (S) pour le repos du bébé présente un mouvement ascendant et descendant (M) ; et
- un côté de confinement (200) qui entoure de manière périphérique la base de repos et de stimulation sensorielle (100) pour le confinement du bébé ;
- des éléments de jonction réutilisables (300) ;
dans lequel ladite base de repos et de stimulation sensorielle (100) et ledit côté de confinement (200) présentent un caractère modulaire permettant la mise en prise et la libération des deux composants (100, 200) par lesdits éléments de jonction réutilisables (300), **caractérisé en ce que** la base de repos et de stimulation sensorielle (100) comprend un matelas de repos (110) sur lequel la surface de repos (S) est située ; et une base de matelas sensorielle (120) sur laquelle le matelas de repos (110) est agencé, et
**en ce que** la base de repos et de stimulation sensorielle (100) comprend également un couvercle enveloppant (140) enveloppant latéralement le matelas de repos (110) et la base de matelas sensorielle (120).

2. Système de confinement et de stimulation sensorielle pour bébés prématurés selon la revendication 1, **caractérisé en ce que** la base de matelas sensorielle (120) consiste en un support de base de matelas (121), délimité au sommet par une feuille (122) agencée sous le matelas de repos (110) en contact avec celui-ci, définissant, entre ledit support de base de matelas (121) et ladite feuille (122), une chambre technique (123) dans laquelle un mécanisme à cames motorisé (130) capable de produire le mouvement ascendant et descendant (M) sur la feuille (122) est situé.

3. Système de confinement et de stimulation sensorielle pour bébés prématurés selon la revendication 2, **caractérisé en ce que** le mécanisme à cames motorisé (130) comprend un premier micro-moteur à engrenages (131) permettant la rotation d'une première came (132) ; et un second micro-moteur à engrenages (133) permettant la rotation d'une seconde came (134) ; dans lequel lesdites cames (132, 134) sont agencées dans une partie centrale de la base de matelas sensorielle (120) en contact avec une partie centrale de la feuille (122).

4. Système de confinement et de stimulation sensorielle pour bébés prématurés selon l'une quelconque des revendications 2 à 4, **caractérisé en ce que** la base de repos et de stimulation sensorielle (100) présente également :
- une première sangle de confinement (141) entourant le côté de confinement (200) au niveau d'une première zone(Z1) de celui-ci, permettant l'enveloppement des bras, de la poitrine ou du dos du bébé ; et
- une seconde sangle de confinement (142), plus large que la première sangle de confinement (141), qui entoure transversalement le côté de confinement (200) au niveau d'une seconde zone (Z2) de celui-ci, permettant l'enveloppement des jambes du bébé.

5. Système de confinement et de stimulation sensorielle pour bébés prématurés selon l'une quelconque des revendications 1 à 4, **caractérisé en ce que** la base de repos et de stimulation sensorielle (100) et le côté de confinement (200) ont en périphérie un contour elliptique correspondant.

6. Système de confinement et de stimulation sensorielle pour bébés prématurés selon l'une quelconque des revendications 1 à 5, **caractérisé en ce que** le côté de confinement (200) présente un profil supérieur ondulé (201) qui définit longitudinalement quatre sections (200a, 200b, 200c, 200d) du côté de confinement (200) :
- un premier segment (200a) dans lequel le profil ondulé (201) présente une première hauteur (h1) configurée pour dépasser la tête du bébé ;
- un deuxième segment (200b) dans lequel le profil ondulé (201) descend de la première hauteur (h1) à une deuxième hauteur (h2) configurée pour laisser le visage du bébé découvert, et monte de ladite deuxième hauteur (h2) à une troisième hauteur (h3) coïncidant avec la première hauteur (h1) ;
- un troisième segment (200c) dans lequel le profil ondulé (201) monte de la troisième hauteur (h3) à une quatrième hauteur (h4) configurée pour couvrir le tronc et les jambes du bébé, et descend de ladite quatrième hauteur (h4) à une cinquième hauteur (h5) coïncidant avec la troisième hauteur (h3) ; et
- un quatrième segment (200d) dans lequel le profil ondulé (201) descend de la cinquième hauteur (h5) à une sixième hauteur (h6) configurée pour permettre au bébé d'étirer ses jambes.

7. Système de confinement et de stimulation sensorielle pour bébés prématurés selon l'une quelconque des revendications 1 à 6, **caractérisé en ce que** les éléments de jonction réutilisables 300 sont choisis parmi des fermetures à glissière, des boutons, des fixations, des crochets et des attaches rapides du type velcro®.

8. Système de confinement et de stimulation sensorielle pour bébés prématurés selon l'une quelconque des revendications 1 à 7, **caractérisé en ce que** les éléments de jonction réutilisables (300) comprennent :
- une première bande de fermeture à glissière (301) suivant le contour supérieur de la base de repos et de stimulation sensorielle (100) ; et
- une seconde bande de fermeture à glissière (302) suivant le contour inférieur du côté de confinement, coopérant avec la première bande de fermeture à glissière (301) pour permettre la mise en prise et la libération des deux composants (100, 200).

9. Système de confinement et de stimulation sensorielle pour bébés prématurés selon l'une quelconque des revendications 1 à 8, **caractérisé en ce qu'**il comprend un premier complément de repos (400) configuré pour être agencé sur la base de repos et de stimulation sensorielle (100) montée de manière interne sur le côté de confinement (200).

10. Système de confinement et de stimulation sensorielle pour bébés prématurés selon la revendication 9, **caractérisé en ce que** le premier complément de repos (400) consiste en :
- une première partie (400a) de contour semi-circulaire, avec une première surface de support (Sa) qui descend jusqu'à une première extrémité (401a), pour supporter la tête du bébé ;
- une deuxième partie (400b) de contour rectangulaire avec une seconde surface de support horizontale (Sb), pour supporter la poitrine du bébé ; et
- une troisième partie (400c) qui se déploie latéralement en deux sous-parties symétriques (401c) avec un évidement incurvé (402c), avec une troisième surface de support (Sc) qui descend dans ledit évidement incurvé (402c), afin de supporter les genoux de l'enfant.

11. Système de confinement et de stimulation sensorielle pour bébés prématurés selon l'une quelconque des revendications 1 à 10, **caractérisé en ce qu'**il comprend un deuxième complément de repos (500) de configuration cylindrique.

12. Système de confinement et de stimulation sensorielle pour bébés prématurés selon l'une quelconque des revendications 1 à 11, **caractérisé en ce qu'**il comprend un troisième complément de repos (600) de configuration cylindrique.

13. Système de confinement et de stimulation sensorielle pour bébés prématurés selon les revendications 11 et 12, **caractérisé en ce que** le deuxième complément de repos (500) et le troisième complément de repos (600) ont des tailles différentes.
